# EUROPEAN PATENT SPECIFICATION

(11) **EP 3 126 915 B1**
(45) Date of publication and mention of the grant of the patent: **18.03.2020**
(21) Application number: 15722581.4
(22) Date of filing: 07.04.2015
(51) Int. Cl.: G04C 17/00, G04B 1/26

(54) **SYSTEMS AND METHODS FOR INDICATING A QUANTITY**
SYSTEME UND VERFAHREN ZUR ANZEIGE EINER MENGE
SYSTÈMES ET PROCÉDÉS POUR INDIQUER UNE QUANTITÉ

(30) Priority: 03.04.2014 US 201461974448 P; 29.04.2014 US 201461985492 P; 06.08.2014 US 201462033686 P
(43) Date of publication of application: 08.02.2017
(73) Proprietor: Preciflex SA, 2000 Neuchâtel (CH)
(72) Inventor: VOUILLAMOZ, Lucien, 8835 Feusisberg (CH); RUFFIEUX, Yves, 1566 St -Aubin (CH)
(74) Representative: Mötteli-Mantelli, Novella
(86) International application number: PCT/IB2015/000446
(87) International publication number: WO 2015/150909

(56) References cited:
- FR-A- 1 552 838
- US-A- 3 783 598
- US-A1- 2009 219 789

## Description

### Background of the Invention

This invention relates to systems and methods for timepieces that include movement/absorption/expansion/contraction of a liquid in a transparent cavity, particularly in wristwatches.

FR1552838A discloses an indication device comprising at least one electrically conductive liquid driven by a pump for conductive liquids, wherein at least one segment of at least one liquid is used as an indicator, which segment the pump drives along adjacent indices of an indicator visible to an observer so as to indicate a quantity to the observer.

Luxury watches exist that indicate time using a meniscus of a liquid which is driven by a purely mechanical system. Such watches are complicated and, consequently, very expensive. A need therefore exists for a low cost, electronic watch that accurately indicates time using the meniscus of a liquid.

### Summary of the Invention

The invention provides an indication device as defined in claim 1. Said indication device includes a channel fillable with one or more liquids. The individual liquids are preferable immiscible with each other. Each individual liquid can be transparent or colored, have the same refractivity as the substrate, can optionally contain solid particles, can be electrical conductive or electrical non-conductive. In a variant, the indication is done with a moving gas bubble, such as a radioactive tritium gas. The channel is formed as a closed loop or in a variant formed with ends ending in a reservoir. An electrically conductive liquid can be moved with the channel by the means of one or more magnetohydrodynamic pumps (MHD pumps). In a further variant, a second liquid is electrical non-conductive or electrically conductive, this liquid is pushed or pulled by the electrically conductive liquid driven by the MHD pump(s).

In a variant, the position of the electrically non-conductive or electrical conductive liquid, in a variant embodied as a gas bubble, within the channel is sensed along the channel by its deviating dielectricity between the two or more liquids. The sensing of the capacitance or the sensing of the change of the capacitance is preferably made by a number of capacitors spread along the channel.

In another variant, the channel is used in a timepiece. The permanent magnets and/or electrodes required in MHD pumps, in order to be non-visible to a user, are incorporated into design/decoration elements or hidden by design/decoration elements. In another variant, the permanent magnets and/or electrodes are visible to the user.

In another variant, the capacitors used to sense the dielectricity or the change of the dielectricity is accomplished with sputtering, preferable as ITO (indium-tin oxide) or FTO (fluorine-doped tin oxide).

In another variant, the channel is formed as a micro capillary.

In another variant, the channel is formed by two or more glass wafers, preferably connected to each other by a suitable bonding process.

In another variant, the channel is formed by two or more polymer wafers, preferably connected to each other by a suitable bonding process.

In another variant, a membrane is embedded between wafers.

In another variant, the channel system has one or more open access holes to allow an initial filling of the system with liquid(s), implicating an automated filling of the system during the production process. Through one access hole, a liquid is inserted, while another access hole provides access to ambient pressure. After initial filling, the access hole(s) are closed in a fluid and/or gas tight manner. Optional, the access hole(s) can be opened and closed again, e.g. for maintenance reasons.

In another variant, as well for a closed loop system, as for a variant with ends ending in a reservoir, is equipped with a system to compensate thermal expansion/contraction of the liquid(s). This is accomplished by a thin and therefore flexible wafer, or a separate gas chamber, or a flexible soft material part, or a membrane. The flexible soft material part can be placed in the channel or in a separate chamber, which is in fluid communication with the channel. The compensation system is non-visible to a user, and in another variant visible to the user. The non-visible system is disposed underneath the visible system.

### Brief Description of the Drawings

**FIG. 1** is a schematic top view of the invention.
**FIG. 2** is a schematic top view of the invention in another variant.
**FIG. 3** is a detail view of an indicator liquid arrangement of the invention.
**FIG. 4** is a schematic perspective view of an MHD motor used in the invention.
**FIG. 5** is a schematic top view of the invention in another variant.
**FIG. 6** is a cross sectional detail view of the liquid reservoir of the invention.
**FIG. 7** is a cross sectional detail view of a variant of the liquid reservoir of the invention.
**FIG. 8** is a cross sectional detail view of another variant of the liquid reservoir of the invention.
**FIG. 9** is a cross sectional view of a detail view of an element of **FIG. 8****.**
**FIG. 10** is a cross sectional detail view of still another variant of the liquid reservoir of the invention.
**FIG. 11** is a schematic top view of the invention in another variant.
**FIG. 12** is a schematic perspective view of the invention in still another variant.
**FIG. 13** is a schematic top view of the invention in a further variant.

Those skilled in the art will appreciate that elements in the Figures are illustrated for simplicity and clarity and have not necessarily been drawn to scale. For example, dimensions may be exaggerated relative to other elements to help improve understanding of the invention and its embodiments. Furthermore, when the terms 'first', 'second', and the like are used herein, their use is intended for distinguishing between similar elements and not necessarily for describing a sequential or chronological order. Moreover, relative terms like 'front', 'back', 'top' and 'bottom', and the like in the Description and/or in the claims are not necessarily used for describing exclusive relative position. Those skilled in the art will therefore understand that such terms may be interchangeable with other terms, and that the embodiments described herein are capable of operating in other orientations than those explicitly illustrated or otherwise described.

### Detailed Description of the Preferred Embodiment

The following description is not intended to limit the scope of the invention which is defined by the attached claims.

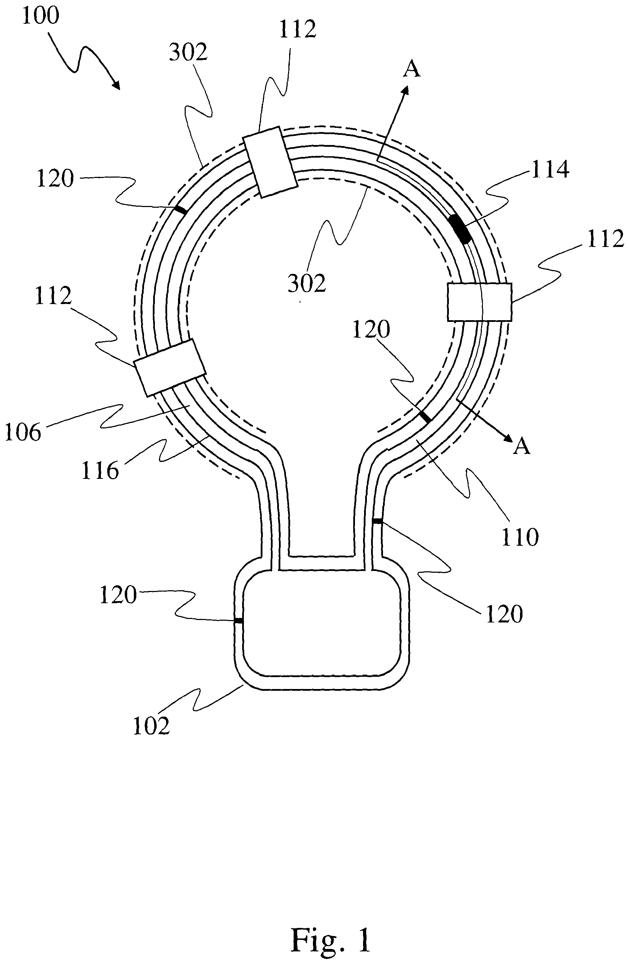

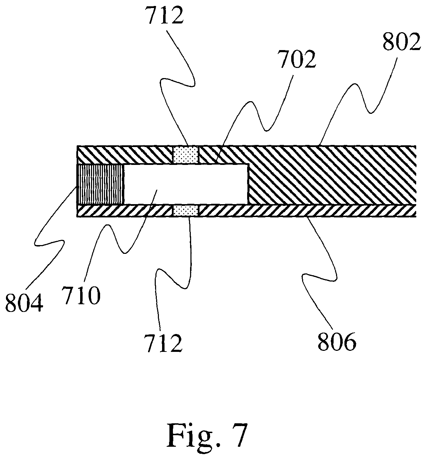

**FIG. 1** is a top view of a system 100 including a capillary channel 116, at its both ends having a reservoir 102 attached. It is appreciated that the capillary channel 116 can take on a variety of geometric cross-sectional two dimensional or three dimensional cross-sectional and overall shapes or configurations, e.g. a cylindrical tube, a square, a rectangle, a circle, an oval, an oval shape, a triangular shape, a pentagonal shape, a hexagonal shape, an octagonal shape, a cubic shape, a spherical shape, an egg shape, a cone shape, a dome shape, a rectangular prism shape, and a pyramidal shape, by way of further example. In this variant the capillary channel 116 is filled with a first essentially electrically conductive, optionally colored liquid 106, implicating for example a Sodium chloride solution and a second electrically conductive or electrically non-conductive, optionally colored liquid 114, implicating for example a silicone oil or a liquid sapphire (as used herein, any liquid having the same refractivity as the substrate), in a variant accomplished using a gas bubble. Of course, the system can contain more or less liquids and another combination of different liquids. Further, this variant is equipped with one or more magnetohydrodynamic pumps (MHD pumps) 112. The channel 116 has optionally one or more open access holes 120 to allow an initial filling of the system with liquid(s), implicating an automated filling of the system during the production process. The system is further equipped with capacitors 302. The system does compensate thermal expansions and compressions of a liquid 106 located in the channel 202, as proposed in **FIGs. 7** to **11****.**

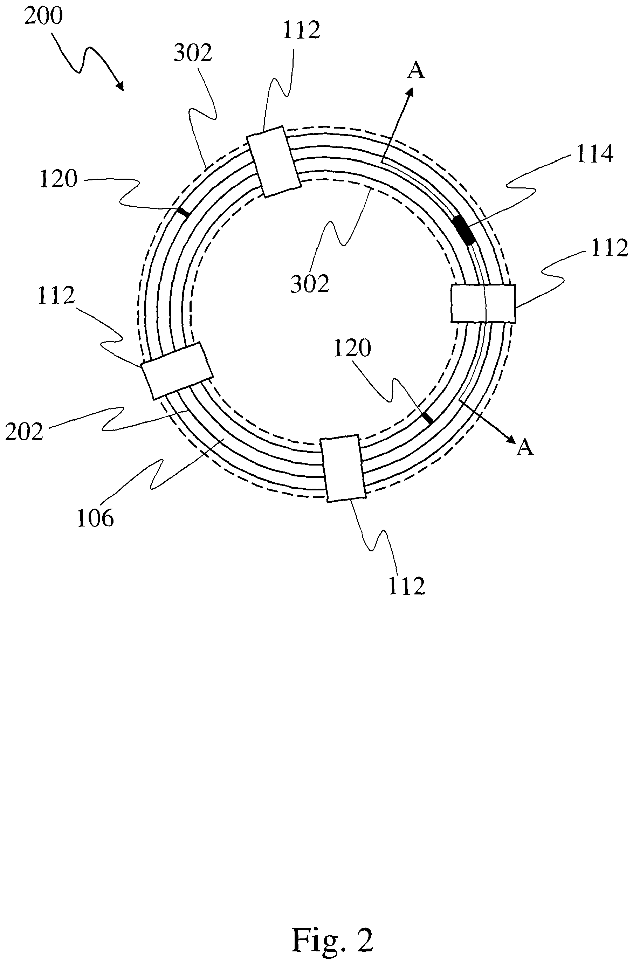

**FIG. 2** is a top view of a system 200 including a capillary channel 202 formed as a closed loop. It is appreciated that the capillary channel 202 can take on a variety of geometric cross-sectional two dimensional or three dimensional cross-sectional and overall shapes or configurations as mentioned above. In this variant the capillary channel 202 is filled with a first essentially electrically conductive, optionally colored liquid 106, implicating for example a Sodium chloride solution and a second electrically conductive or electrically non-conductive, optionally colored liquid 114, implicating for example a silicone oil or liquid sapphire, in a variant accomplished using a gas bubble. Of course, the system can contain more or less liquids and another combination of different liquids. Further, this variant is equipped with one or more magnetohydrodynamic pumps (MHD pumps) 112. The channel 202 has optionally one or more open access holes 120 to allow an initial filling of the system with liquid(s), implicating an automated filling of the system during the production process. The system is further equipped with capacitors 302. The system does compensate thermal expansions and compressions of a liquid 106 located in the channel 202, as proposed in **FIGs. 7** to **11****.**

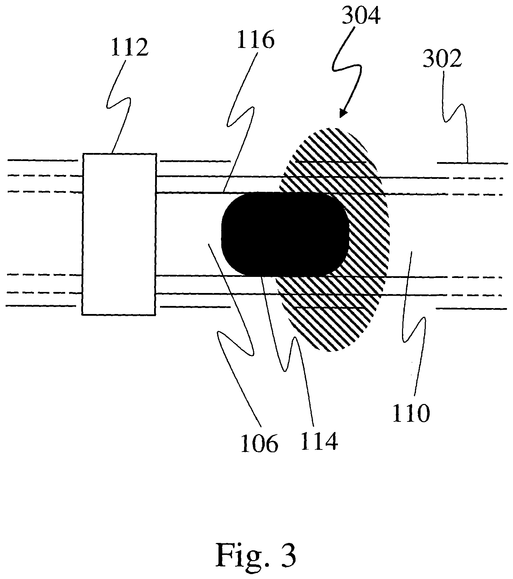

**FIG. 3** is a sectional view A-A of Fig.1 including a capillary channel 116. In this variant the capillary channel 116 is filled with a first essentially electrically conductive, optionally colored liquid 106, implicating for example a Sodium chloride solution and a second electrically conductive or electrically non-conductive, optionally colored liquid 114, implicating for example a silicone oil or liquid sapphire, and in a variant accomplished using a gas bubble. Of course, the system can contain more or less liquids and another combination of different liquids. Further, this variant is equipped with one or more magnetohydrodynamic pumps (MHD pumps) 112 to drive an electrically conductive or a non-conductive, optionally colored liquid 114, implicating for example a silicone oil or liquid sapphire, in a variant accomplished using a gas bubble, surrounded by an optionally colored, transparent conductive liquid 110. The system is further equipped with capacitors 302 used to sense the dielectricity or the change of the dielectricity essentially at areas 304 near the capacitor or the pair of capacitors or the triple of capacitors. The capacitors are made by sputtering, preferable as ITO (indium-tin oxide) or FTO (fluorine-doped tin oxide). Several capacitors are placed along the channel 116. The dielectricity and/or the change of dielectricity can be sensed by dedicating one, a pair or a triple of capacitors to an area 304.

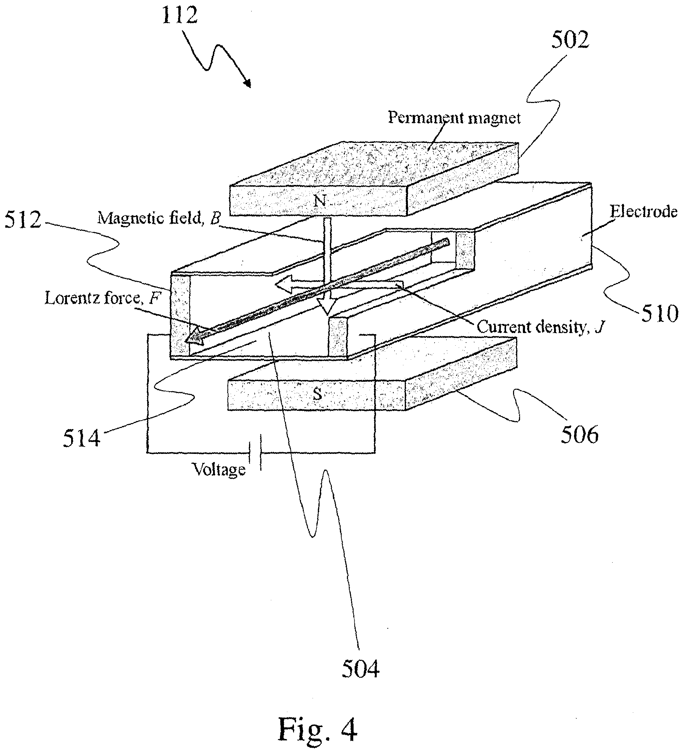

**FIG. 4** is a perspective view of a magnetohydrodynamic pumps (MHD pumps) 112. The MHD pump 112 includes a permanent magnet with its polarization North 502 directed towards a channel 504, a permanent magnet with its polarization South 506 directed towards a channel 504 and essentially opposite to permanent magnet with its polarization North 502. The channel contains liquids 514, implicating for example a silicone oil, liquid sapphire or a Sodium chloride solution, in a variant accomplished using a gas bubble. The system is further equipped with a pair of electrodes 510, 512, reframing the channel 504 and essentially 90° to the permanent magnets 502, 506. To the electrodes 510, 512 a direct current (DC), positive or negative polarized, can be applied. The swap of polarization will reverse the flow of the liquids 514. The permanent magnets 502, 506 may either be in contact with the liquids 514 or not be in contact with the liquids 514 and/or gas. The electrodes 510, 512 are in contact with the liquids 514 and/or gas.

Considering the circular capillary sub-systems 100 or 200, and its various dimensions, a time of 60 seconds is used to completely fill the circular capillary sub-system 100 or 200. An exemplary specification for a robust, efficient, fit for purpose MHD pump 112 is as follows:

| | |
|---|---|
| 1. Capillary sub-system 100 or 200 cross-sectional area: | A= 0.5 mm² |
| 2. MHD flow mean velocity: | V_{MHD} = 1.895 mm/s |
| 3. MHD flow rate: | Q_{MHD} = 57.165 µL/min |

Of course, the stronger the MHD pump 112 is the more fluid is moved into cavity 116 or 202 at a faster rate. Slower rates of filling are accomplished by weaker MHD pumps 112 depending on their overall specifications and pumping strength.

Now looking at other MHD pump variants in the comparison provided below, and summarized in Table 1 below, it is appreciated that the example highlighted in red approximates the required specifications. Other MHD pumps can be used, depending upon the requirements of fluid movement, either continuous or intermittent, or those that require faster or slower fluid movement in the cavity 116 or 202. It is appreciated that an MHD pump 112, and circular capillary sub-system 100 or 200 featuring cavity 116 or 202 is provided in another variant. Other variants of dimensions (area, volume, geometric shape) of components of sub-system 100 or 200 are also provided in combination with other MHD pumps that have other engineered properties and modes of operation, some being fit for purpose and some not, but preferably, the specifications of MHD pump 112 framed in Table 1 are preferable for optimal fluid movement in cavity 116 or 202.

**Table 1**

| | U | I | A | A*_{J}* | *I* | J | B | ΔP*_{MHD}* | *V_{MHD}* | Q*_{MHD}* |
|---|---|---|---|---|---|---|---|---|---|---|
| | (V) | (mA) | (mm²) | (mm²) | (mm) | (A·m⁻²) | (T) | (Pa) | (mm·s⁻¹) | (*µ*L·min⁻¹) |
| Jang *et al*.[1] | 30 DC | 1.8 | 0.4 | 30 | 30 | 60 | 0.44 | 1 | 2.6* | 63* |
| Leventis *et al*. [2] | >1.3 DC | 35 | 18 | 225 | 75 | 155 | 1.35 | 16 | 0.4 | 450·10³ |
| Bau *et al.* [3] | 4 DC | 15 | 1.9 | 292 | 172 | 51 | 0.4 | 3.5 | 0.4 | 45 |
| Lemoff *et al.* [4] | 6.6 AC | 140 | 0.2 | 1.5 | 4 | 92105 | 0.013 | 5 | 1.5 | 18 |
| West *et al.* [5] | 5 AC | 90 | 0.2 | 5 | 28 | 17684 | 0.011 | 5.5 | 0.24 | 3 |
| Eijkel *et al.* [6] | 4 AC | 40 | 6.10⁻³ | 2 | 63 | 21100 | 0.1 | 133 | 0.04 | 14·10⁻³ |
| | | | | | | | | | | |
| Chapter 4 | 16 DC | 4.8 | 8.8·10⁻³ | 1.2 | 16 | 4000 | 0.42 | 27 | 0.5 | 0.3 |
| Chapter 6 | 19 DC | 2 | 8.8·10⁻³ | 1.2 | 16 | 1600 | 7.05 | 180 | 2.8 | 1.5 |

| | | | | | | | | | | |
|---|---|---|---|---|---|---|---|---|---|---|
| Performance comparison of previously published MHD pumps with our MHD pump presented in Ch 4 and 6. All values for voltage (U), current (I), channel cross-sectional area (A), total length of electrodes along the pumping channel (*I*). MHD flow mean velocity in the pumping channel (v*_{MHD}*) and MHD flow rate (Q*_{MHD}*) were experimental data, and were taken from references [1-6]. Most of the values for the electrode cross-sectional area (A*_{J}*) and current density (J) across the pumping channel had to be calculated. The body force (ΔP*_{MHD}*) generated by the pumps was calculated thanks to relation 2.14. **Both values wen taken from experimental measurement. If calculated with relations 2.16 and 2.15, the predicted velocity and flow rate would be 0.16 mm·s*⁻¹ *and 4 µL·min*⁻¹ *respectively*. | | | | | | | | | | |

The following list of references showing the variety of MHD pumps in the market:
1. Design, Microfabrication, and Characterization of MHD Pumps and their Applications in NMR Environments, Thesis by Alexandra Homsy, 2006, the content of which is incorporated herein by reference thereto.
2. Bislug Flow in Circular and Noncircular Channels and the Role of Interface Stretching on Energy Dissipation, Thesis by Joseph E. Hernandez, August 2008, the content of which is incorporated herein by reference thereto.

In yet a further aspect, the invention also provides for a grouping of sub-systems that include a circular (or other geometric configuration) capillary sub-system(s) with one or more MHD pumps 112. The groups include one or more MHD pumps 112 and tube/cavity combinations or groups of inter-related sub-systems. The one or more than one MHD pump 112 manages displacement of one or more fluids within individual circular capillary sub-systems or by way of manifold into more than one capillary sub-systems, in series or in parallel, alone or in combination with other MHD pumps providing for multiple indicator functionality within a single device, e.g. a wristwatch.

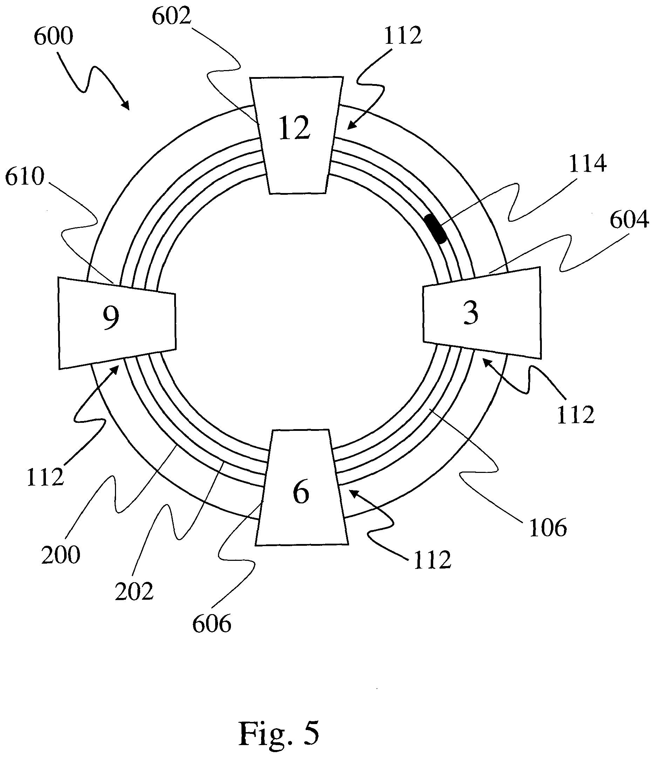

**FIG. 5** is a perspective view of a timepiece 600 equipped with system 200. The system 200 includes a capillary channel 202 formed as a closed loop. In this variant the capillary channel 202 is filled with a first essentially electrically conductive liquid 106, implicating for example a Sodium chloride solution and a second electrically conductive or electrically non-conductive, optionally colored liquid 114, implicating for example silicone oil or liquid sapphire, in a variant accomplished using a gas bubble. Of course, the system can contain more or less liquids and another combination of different liquids. Further, this variant is equipped with four magnetohydrodynamic pumps (MHD pumps) 112. The magnetohydrodynamic pumps (MHD pumps) are incorporated into indices/design/decoration elements or hidden by indices/design/decoration elements 602, 604, 606, 610, in order to be non-visible to a user.

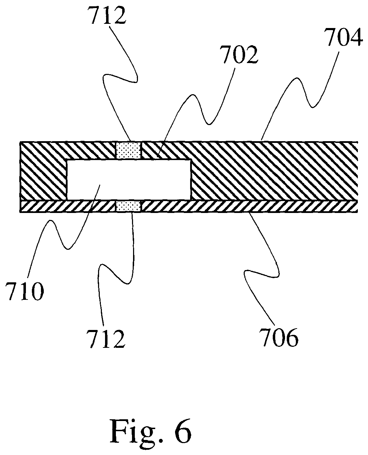

**FIG. 6** is a cross sectional view of variant of system 100 or system 200. The channel 702 is formed by two wafers 704, 706, implicating wafers made out of glass and/or polymer. The wafers 704, 706 are fixed to each other preferably by a suitable bonding process. The channel 702 contains one or more liquids and/or gas 710, implicating for example a silicone oil, liquid sapphire or a Sodium chloride solution. Wafer 706 is particularly thin in the region of the channel 702 and is therefore enough flexible in that region to compensate thermal expansions and compressions of a liquid 710 located in the channel 702. The channel 702 has optionally one or more open access holes 712 to allow an initial filling of the system with liquid(s) 710, implicating an automated filling of the system during the production process.

**FIG. 7** is a cross sectional view of variant of system 100 or system 200. The channel 702 is formed by three or more wafers 802, 804, 806, implicating wafers made out of glass and/or polymer. The wafers 802, 804, 806 are fixed to each other preferably by a suitable bonding process. The channel 702 contains one or more liquids and/or gas 710, implicating for example a silicone oil, liquid sapphire or a Sodium chloride solution. Wafer 806 is particularly thin in the region of the channel 702 and is therefore enough flexible in that region to compensate thermal expansions and compressions of a liquid 710 located in the channel 702. The channel 702 has optionally one or more open access holes 712 to allow an initial filling of the system with liquid(s) 710, implicating an automated filling of the system during the production process.

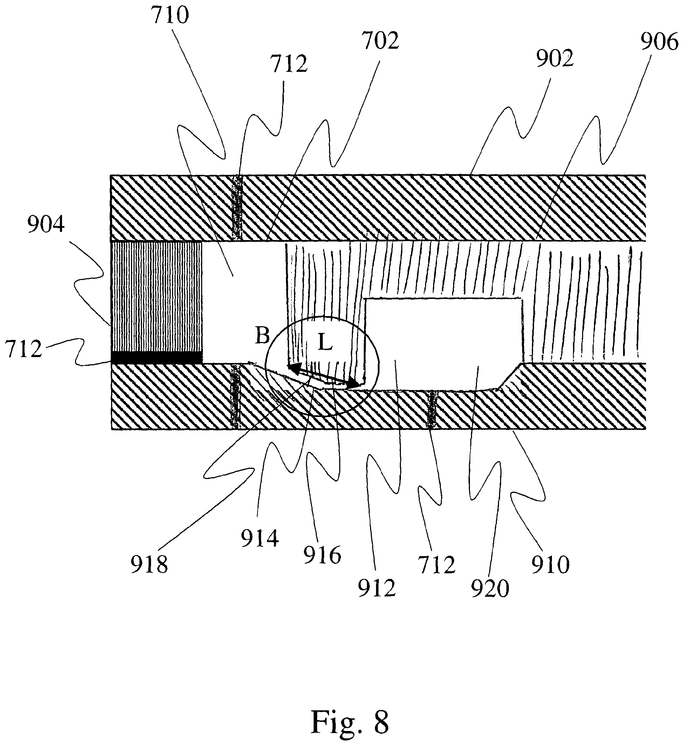

**FIG. 8** is a cross sectional view of variant of system 100 or system 200. The channel 702 is formed by four wafers 902, 904, 906, 910, implicating wafers made out of glass and/or polymer. The system can also be formed by less or more wafers. The wafers 902, 904, 906, 910 are fixed to each other preferably by a suitable bonding process. The channel 702 contains one or more liquids 710, implicating for example a silicone oil, liquid sapphire or a Sodium chloride solution. Wafers 906, 910 form a gas chamber 912 containing essentially gas 920. Gas chamber 912 and channel 702 are connected to each other through a thin transit passage 914. The thin transit passage has a certain length 916, typically 0.5-2mm. The intersection 918 between gas 920 and liquid 710 is essentially within the length 916. The compressibility of gas 920 in combination with this system allows to compensate thermal expansions and compressions of a liquid 710 located in the channel 702. The channel 702 and/or the gas chamber 912 has optionally one or more open access holes 712 to allow an initial filling of the system with liquid(s) 710 and/or gas 920, implicating an automated filling of the system during the production process.

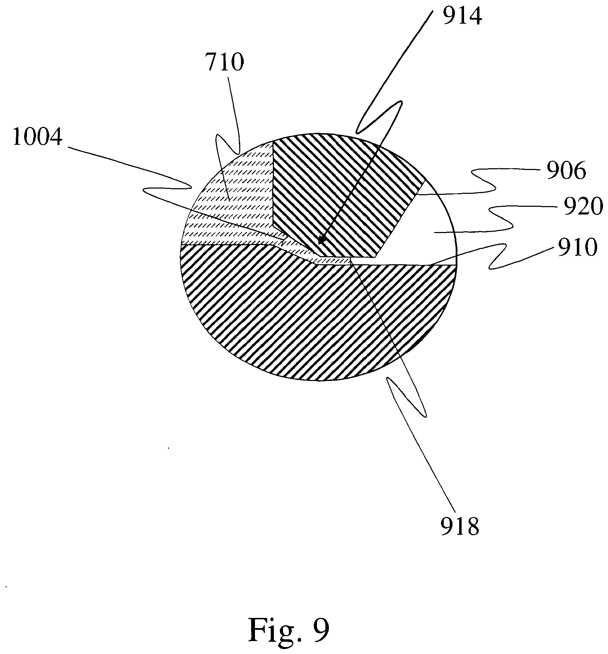

**FIG. 9** is the detail view B of **FIG. 8****.** The thin transit passage 914 is shown in detail. To optimize the trapping of a liquids 710, the angle 1004 between wafers 906, 910 at the entrance of the thin transit passage can be positive, zero or negative. The forming of the thin transit passage 914 can further be freely chosen in order to optimize a proper separation of gas 920 and liquid 710. To prevent mixing or migration of gas 920 from gas chamber 912 to the channel 702, the dimensions and shape of the thin transit passage 914 has to be adapted according to the viscosities of the liquids 710.

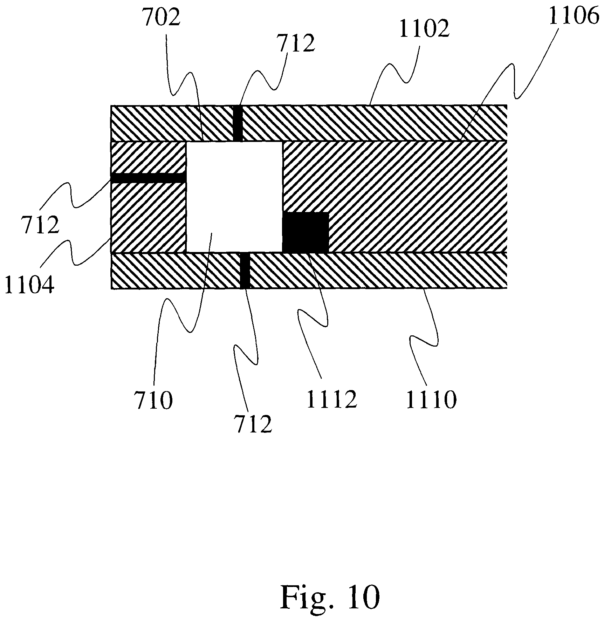

**FIG. 10** is a cross sectional view of variant of system 100 or system 200. The channel 702 is formed by four wafers 1102, 1104, 1106, 1110, implicating wafers made out of glass and/or polymer. The system can also be formed by less or more wafers. The wafers 1102, 1104, 1106, 1110 are fixed to each other preferably by a suitable bonding process. The channel 702 contains one or more liquids 710, implicating for example a silicone oil, liquid sapphire or a Sodium chloride solution, in a variant accomplished using a gas bubble. A soft material 1112 is located at a specific place to be in contact with the liquid and/or gas 710. The soft material 1112 has the property to compensate thermal expansions and compressions of a liquid 710 located in the channel 702. The channel 702 has optionally one or more open access holes 712 to allow an initial filling of the system with liquid(s) and or gas 710, implicating an automated filling of the system during the production process.

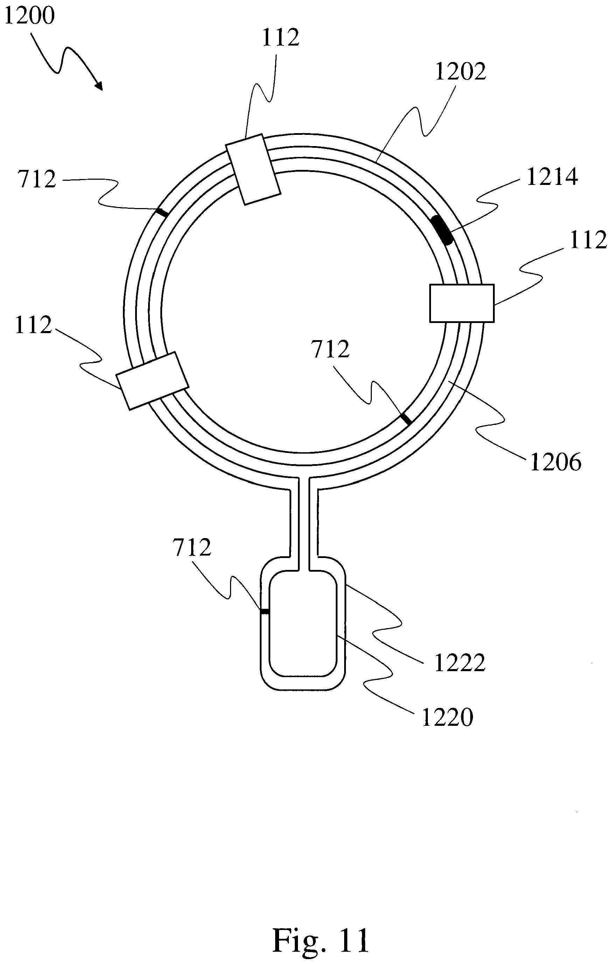

**FIG. 11** is a top view of a system 1200 including a capillary channel 1202 formed as a closed loop. It is appreciated that the capillary channel 1202 can take on a variety of geometric cross-sectional two dimensional or three dimensional cross-sectional and overall shapes or configurations. In this variant the capillary channel 1202 is filled with a first essentially electrically conductive, optionally colored liquid 1206, implicating for example a Sodium chloride solution and a second electrically conductive or electrically non-conductive, optionally colored liquid 1214, implicating for example a silicone oil or liquid sapphire, in a variant accomplished using a gas bubble. Of course, the system can contain more or less liquids and another combination of different liquids. Further, this variant is equipped with one or more magnetohydrodynamic pumps (MHD pumps) 112. A reservoir 1220 is located at a specific place in fluid communication with the channel 1202. The housing 1222 of the reservoir 1220 has the ability to compensate thermal expansions and compressions of a liquid 1206 located in the channel 1202. Such compensation, however, may also be obtained such as described in FIG. 3 of PCT/2015/ 150910, filed 7 April 2015, entitled SYSTEMS AND METHODS FOR ABSORBTION/EXPANSION/CONTRACTION/MOVEMENT OF. A LIQUID IN A TRANSPARENT CAVITY. The channel 1202 and/or the housing 1222 of the reservoir 1220 has optionally one or more open access holes 712 to allow an initial filling of the system with liquid(s) or gas 1206, 1214, implicating an automated filling of the system during the production process.

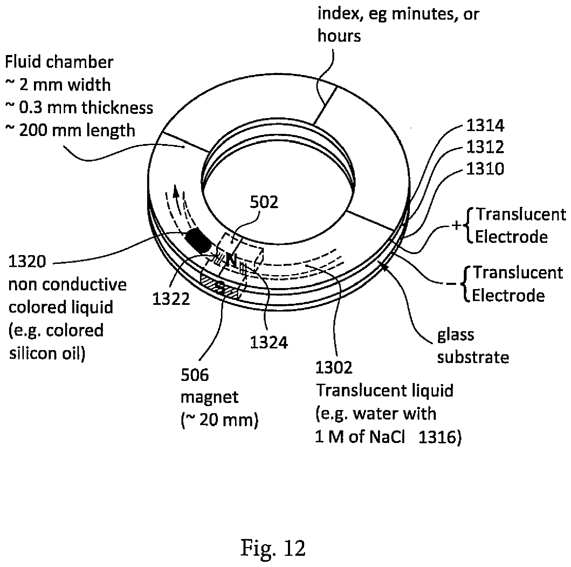

**FIG. 12** is a variant of a system as e.g. described in **Fig.2****,** **Fig. 5** or **Fig.11****,** including a closed loop 1302. The channel 1306 is formed by fixing two or more wafers 1310, 1312, 1314 together, implicating wafers made out of glass and/or polymer. The channel 1306 may be filled with fluid, gas, solid particles or a combination thereof. In this variant, the channel is filled with two different types of fluids 1316, 1320, implicating for example a silicone oil, liquid sapphire or a Sodium chloride solution. At least one of the filled liquids is essentially electrically conductive. An MHD pump 112 is integrated having its permanent magnets 502, 506 placed along the inner diameter and along the outer diameter between two wafers 1310, 1314. Further, wafer 1310 and wafer 1314 are electrically conductive and function as electrodes. The electrical conductivity on wafers 1310, 1314 are preferable achieved by sputtering, preferable as ITO (indium-tin oxide) or FTO (fluorine-doped tin oxide). The essentially electrically conductive liquid 1316 will be driven forward or backwards by a Lorenz force, created by the magnetic field 1322 generated by the permanent magnets 502, 506 in combination with the electrical field 1324 generated between the two wafers 1310, 1314 connected to a direct current (DC) voltage source. The swap of polarization will reverse the flow of the liquids 1316, 1320. Of course, this variant contains mechanism to compensate thermal expansion and/or contractions of the fluid, as described before. And of course, this variant contains capacitors to measure the dielectricity and/or the change of dielectricity as described in **Fig.3****.**

Reference is made to the provisional US patent application Serial No. 61/787,727, filed on 15 March 2013, and the International patent application no. PCT/IB2014/000373, filed on 17 March 2014, both entitled "TEMPERATURE DRIVEN WINDING SYSTEM".

Multiple variations and modifications are possible in the embodiments of the invention described here. Although certain illustrative embodiments of the invention have been shown and described here, a wide range of changes, modifications, and substitutions is contemplated in the foregoing disclosure. While the above description contains many specific details, these should not be construed as limitations on the scope of the invention, but rather exemplify one or another preferred embodiment thereof. In some instances, some features of the present invention may be employed without a corresponding use of the other features. Accordingly, it is appropriate that the foregoing description be construed broadly and understood as being illustrative only, the scope of the invention being limited only by the claims which ultimately issue in this application.

## Claims

1. An indication device comprising at least one electrically conductive liquid (106, 110, 114, 1206, 1214, 1316), a rigid chamber (116, 202, 1202), and a pump (112), said at least one electrically conductive liquid being enclosed in said rigid chamber, wherein at least one segment of at least one liquid (106, 110, 114, 1206, 1214, 1316) is an indicator (600), which segment the pump (112) drives along an adjacent set of indices (602, 604, 606, 610) of an indicator visible to an observer so as to indicate a quantity, **characterized in that** said pump is a magneto-hydrodynamic pump (112) for conductive liquids (106, 110, 114, 1206, 1214, 1316).

2. The device of claim 1, wherein further the at least one liquid (106, 110, 114, 1206, 1214, 1316) is enclosed in said rigid chamber (116, 202, 1202) of a closed loop (1302) that has at least one exposed, at least partially transparent surface allowing the observer to observe the position of the at least one segment of the liquid (106, 110, 114, 1206, 1214, 1316, 514, 710, 920, 1320), and wherein the device further includes a mechanism accommodating thermal expansion or contraction of the liquids, the mechanism disposed so as to be substantially invisible to the observer.

3. The device of claim 2, wherein at least one liquid (106, 110, 114, 1206, 1214, 1316) is a colored liquid, has a suspended particulate therein, or has the same refractive index as the rigid chamber.

4. The device of claim 2, wherein a meniscus of the segment of the at least one liquid is sensed (302) and controlled to position the meniscus at a desired position to ensure accurate indication of the quantity.

5. The device of claim 4, wherein capacitance (302) is used to detect the position of the segment of the at least one liquid (106, 110, 114, 1206, 1214, 1316), so as to enable control thereof.

6. The device of claim 4, wherein conductance (1310, 1314) is used to detect the position of segment of the at least one liquid (106, 110, 114, 1206, 1214, 1316), so as to enable control thereof.

7. The device of claim 2, wherein at least two magneto-hydrodynamic pumps (112) are spaced apart along the elongated chamber so as to ensure that at any operational position of the liquid (106, 110, 114, 1206, 1214, 1316), the liquid (106, 110, 114, 1206, 1214, 1316) can be pumped.

8. The device of claim 1, wherein the direction of motion of the liquid (106, 110, 114, 1206, 1214, 1316, 514, 710, 920, 1320) is changed by changing the polarity of the magneto-hydrodynamic pumps (112).

9. The device of claim 1, wherein the device has a mechanism accommodating thermal expansion and/or contraction, which is a gas-filled chamber (912) portion of the rigid chamber connected to the liquid-filled portion of the rigid chamber by a passageway (914) portion of the rigid chamber.

10. The device of claim 1, wherein at least one liquid (106, 110, 114, 1206, 1214, 1316) is a colored liquid.

11. The device of claim 1, in which the at least one liquid (106, 110, 114, 1206, 1214, 1316) has the same refractive index as the rigid chamber.

12. The device of claim 1, wherein the at least one liquid (106, 110, 114, 1206, 1214, 1316) has a suspended particulate.

13. The device of claim 1, wherein a meniscus of the segment of the at least one liquid is sensed (302) and controlled to position the meniscus at a desired position to ensure accurate indication of the quantity.

14. The device of claim 1, wherein at least two magneto-hydrodynamic pumps (112) are spaced apart along the elongated chamber so as to ensure that at any operational position of the liquid (106, 110, 114, 1206, 1214, 1316), the liquid (106, 110, 114, 1206, 1214, 1316) can be pumped.

15. The device of claim 14, wherein the direction of motion of the liquid (106, 110, 114, 1206, 1214, 1316, 514, 710, 920, 1320) is changed by changing the polarity of the magneto-hydrodynamic pumps (112).

## Patentansprüche

1. Indikationsvorrichtung, umfassend mindestens eine elektrisch leitfähige Flüssigkeit (106, 110, 114, 1206, 1214, 1316), eine starre Kammer (116, 202, 1202) und eine Pumpe (112), wobei die mindestens eine elektrisch leitfähige Flüssigkeit in der starren Kammer enthalten ist, wobei mindestens ein Segment von mindestens einer Flüssigkeit (106, 110, 114, 1206, 1214, 1316) ein Indikator (600) ist, wobei die Pumpe (112) das Segment an einem benachbarten Satz von Indizes (602, 604, 606, 610) eines Indikators entlang treibt, der für einen Beobachter sichtbar ist, um eine Quantität anzuzeigen,
**dadurch gekennzeichnet, dass** die Pumpe eine magnetohydrodynamische Pumpe (112) für leitfähige Flüssigkeiten (106, 110, 114, 1206, 1214, 1316) ist.

2. Vorrichtung nach Anspruch 1, wobei weiter die mindestens eine Flüssigkeit (106, 110, 114, 1206, 1214, 1316) in der starren Kammer (116, 202, 1202) eines geschlossenen Kreislaufs (1302) enthalten ist, die mindestens eine exponierte, zumindest teilweise transparente Oberfläche aufweist, die es dem Beobachter ermöglicht, die Position des mindestens einen Segments der Flüssigkeit (106, 110, 114, 1206, 1214, 1316, 514, 710, 920, 1320) zu beobachten, und wobei die Vorrichtung weiter einen Mechanismus aufweist, der eine thermische Expansion oder Kontraktion der Flüssigkeiten ermöglicht, wobei der Mechanismus derart angeordnet ist, dass er für den Beobachter im Wesentlichen unsichtbar ist.

3. Vorrichtung nach Anspruch 2, wobei mindestens eine Flüssigkeit (106, 110, 114, 1206, 1214, 1316) eine gefärbte Flüssigkeit ist, ein suspendiertes Partikelmaterial darin aufweist oder den gleichen Brechungsindex wie die starre Kammer aufweist.

4. Vorrichtung nach Anspruch 2, wobei ein Meniskus des Segments der mindestens einen Flüssigkeit erfasst (302) und gesteuert wird, um den Meniskus an einer gewünschten Position zum Sicherstellen einer präzisen Anzeige der Quantität zu positionieren.

5. Vorrichtung nach Anspruch 4, wobei Kapazitanz (302) zum Detektieren der Position des Segments der mindestens einen Flüssigkeit (106, 110, 114, 1206, 1214, 1316) verwendet wird, um eine Steuerung davon zu ermöglichen.

6. Vorrichtung nach Anspruch 4, wobei Konduktanz (1310, 1314) zum Detektieren der Position des Segments der mindestens einen Flüssigkeit (106, 110, 114, 1206, 1214, 1316) verwendet wird, um eine Steuerung davon zu ermöglichen.

7. Vorrichtung nach Anspruch 2, wobei mindestens zwei magnetohydrodynamische Pumpen (112) entlang der länglichen Kammer voneinander beabstandet sind, um sicherzustellen, dass die Flüssigkeit (106, 110, 114, 1206, 1214, 1316) an jeder Betriebsposition der Flüssigkeit (106, 110, 114, 1206, 1214, 1316) gepumpt werden kann.

8. Vorrichtung nach Anspruch 1, wobei die Bewegungsrichtung der Flüssigkeit (106, 110, 114, 1206, 1214, 1316, 514, 710, 920, 1320) durch Ändern der Polarität der magnetohydrodynamischen Pumpen (112) geändert wird.

9. Vorrichtung nach Anspruch 1, wobei die Vorrichtung einen Mechanismus, der eine thermische Expansion und/oder Kontraktion ermöglicht, aufweist, welcher ein gasgefüllter Kammerabschnitt (912) der starren Kammer ist, der mit dem flüssigkeitsgefüllten Abschnitt der starren Kammer durch einen Kanalabschnitt (914) der starren Kammer verbunden ist.

10. Vorrichtung nach Anspruch 1, wobei mindestens eine Flüssigkeit (106, 110, 114, 1206, 1214, 1316) eine gefärbte Flüssigkeit ist.

11. Vorrichtung nach Anspruch 1, wobei die mindestens eine Flüssigkeit (106, 110, 114, 1206, 1214, 1316) den gleichen Brechungsindex wie die starre Kammer aufweist.

12. Vorrichtung nach Anspruch 1, wobei die mindestens eine Flüssigkeit (106, 110, 114, 1206, 1214, 1316) ein suspendiertes Partikelmaterial aufweist.

13. Vorrichtung nach Anspruch 1, wobei ein Meniskus des Segments der mindestens einen Flüssigkeit erfasst (302) und gesteuert wird, um den Meniskus an einer gewünschten Position zum Sicherstellen einer präzisen Anzeige der Quantität zu positionieren.

14. Vorrichtung nach Anspruch 1, wobei mindestens zwei magnetohydrodynamische Pumpen (112) entlang der länglichen Kammer voneinander beabstandet sind, um sicherzustellen, dass die Flüssigkeit (106, 110, 114, 1206, 1214, 1316) an jeder Betriebsposition der Flüssigkeit (106, 110, 114, 1206, 1214, 1316) gepumpt werden kann.

15. Vorrichtung nach Anspruch 14, wobei die Bewegungsrichtung der Flüssigkeit (106, 110, 114, 1206, 1214, 1316, 514, 710, 920, 1320) durch Ändern der Polarität der magnetohydrodynamischen Pumpen (112) geändert wird.

## Revendications

1. Dispositif d'indication comprenant au moins un liquide électriquement conducteur (106, 110, 114, 1206, 1214, 1316), une chambre rigide (116, 202, 1202) et une pompe (112), ledit au moins un liquide électriquement conducteur étant enfermé dans ladite chambre rigide,
au moins un segment d'au moins un liquide (106, 110, 114, 1206, 1214, 1316) étant un indicateur (600), segment que la pompe (112) entraîne le long d'un ensemble adjacent de repères (602, 604, 606, 610) d'un indicateur visible par un observateur de façon à indiquer une quantité,
**caractérisé par le fait que** ladite pompe est une pompe magnétohydrodynamique (112) pour liquides conducteurs (106, 110, 114, 1206, 1214, 1316).

2. Dispositif selon la revendication 1, dans lequel en outre l'au moins un liquide (106, 110, 114, 1206, 1214, 1316) est enfermé dans ladite chambre rigide (116, 202, 1202) d'une boucle fermée (1302) qui a au moins une surface exposée, au moins partiellement transparente, permettant à l'observateur d'observer la position de l'au moins un segment du liquide (106, 110, 114, 1206, 1214, 1316, 514, 710, 920, 1320), et le dispositif comprenant en outre un mécanisme s'adaptant à la dilatation thermique ou contraction thermique des liquides, le mécanisme étant disposé de façon à être sensiblement invisible à l'observateur.

3. Dispositif selon la revendication 2, dans lequel au moins un liquide (106, 110, 114, 1206, 1214, 1316) est un liquide coloré, a des particules en suspension à l'intérieur de celui-ci ou a le même indice de réfraction que la chambre rigide.

4. Dispositif selon la revendication 2, dans lequel un ménisque du segment de l'au moins un liquide est détecté (302) et contrôlé pour positionner le ménisque dans une position souhaitée pour assurer une indication précise de la quantité.

5. Dispositif selon la revendication 4, dans lequel une capacité (302) est utilisée pour détecter la position du segment de l'au moins un liquide (106, 110, 114, 1206, 1214, 1316), de façon à permettre un contrôle de celle-ci.

6. Dispositif selon la revendication 4, dans lequel une conductance (1310, 1314) est utilisée pour détecter la position du segment de l'au moins un liquide (106, 110, 114, 1206, 1214, 1316), de façon à permettre un contrôle de celle-ci.

7. Dispositif selon la revendication 2, dans lequel au moins deux pompes magnéto-hydrodynamiques (112) sont espacées le long de la chambre allongée de façon à assurer que, dans n'importe quelle position opérationnelle du liquide (106, 110, 114, 1206, 1214, 1316), le liquide (106, 110, 114, 1206, 1214, 1316) peut être pompé.

8. Dispositif selon la revendication 1, dans lequel la direction de mouvement du liquide (106, 110, 114, 1206, 1214, 1316, 514, 710, 920, 1320) est changée par changement de la polarité des pompes magnéto-hydrodynamiques (112).

9. Dispositif selon la revendication 1, dans lequel le mécanisme s'adaptant à la dilatation thermique et/ou la contraction thermique est une partie de chambre remplie de gaz (912) de la chambre rigide reliée à la partie remplie de liquide de la chambre rigide par une partie de passage (914) de la chambre rigide.

10. Dispositif selon la revendication 1, dans lequel au moins un liquide (106, 110, 114, 1206, 1214, 1316) est un liquide coloré.

11. Dispositif selon la revendication 1, dans lequel l'au moins un liquide (106, 110, 114, 1206, 1214, 1316) a le même indice de réfraction que la chambre rigide.

12. Dispositif selon la revendication 1, dans lequel l'au moins un liquide (106, 110, 114, 1206, 1214, 1316) a une matière particulaire en suspension.

13. Dispositif selon la revendication 1, dans lequel un ménisque du segment de l'au moins un liquide est détecté (302) et contrôlé pour positionner le ménisque dans une position souhaitée pour assurer une indication précise de la quantité.

14. Dispositif selon la revendication 1, dans lequel au moins deux pompes magnéto-hydrodynamiques (112) sont espacées le long de la chambre allongée de façon à assurer que, dans n'importe quelle position opérationnelle du liquide (106, 110, 114, 1206, 1214, 1316), le liquide (106, 110, 114, 1206, 1214, 1316) peut être pompé.

15. Dispositif selon la revendication 14, dans lequel la direction de mouvement du liquide (106, 110, 114, 1206, 1214, 1316, 514, 710, 920, 1320) est changée par changement de la polarité des pompes magnéto-hydrodynamiques (112).
